# FASCICULE DE BREVET EUROPEEN

(11) **EP 3 827 243 B1**
(45) Date de publication et mention de la délivrance du brevet: **28.06.2023**
(21) Numéro de dépôt: 19762716.9
(22) Date de dépôt: 25.07.2019
(51) Int. Cl.: G01N 3/303, G01N 3/30, A61B 5/00, A61B 17/92, A61F 2/46, A61B 17/16

(54) **DISPOSITIF POUR EVALUER LA SOLIDITE D'UN MATERIAU**
VORRICHTUNG ZUR BEWERTUNG DER HÄRTE EINES MATERIALS
DEVICE FOR EVALUATING HARDNESS OF A MATERIAL

(30) Priorité: 26.07.2018 FR 1856960
(43) Date de publication de la demande: 02.06.2021
(73) Titulaire: Centre national de la recherche scientifique, 75016 Paris (FR); Université Paris XII Val de Marne, 94010 Créteil Cedex (FR)
(72) Inventeur: HAIAT, Guillaume, 94150 Rungis (FR); HUBERT, Alexis, 94140 Alfortville (FR)
(74) Mandataire: Osha Liang
(86) Numéro de dépôt international: PCT/EP2019/070131
(87) Numéro de publication internationale: WO 2020/021046

(56) Documents cités:
- EP-A1- 2 923 677
- US-A- 2 955 456

## Description

### DOMAINE TECHNIQUE

L'invention concerne un dispositif pour évaluer la solidité d'un matériau. Un tel dispositif peut être utilisé, notamment, pour évaluer la solidité d'un tissu comme par exemple l'os ou le cartilage lors d'une opération chirurgicale, en particulier lors d'une ostéotomie.

### ARRIERE PLAN

Une ostéotomie est une opération chirurgicale consistant à sectionner, fracturer ou façonner un os ou un cartilage, dans le but de remédier à une difformité. Elle se pratique généralement à l'aide d'un ciseau chirurgical de forme adaptée, appelé ostéotome, et d'un maillet. Le praticien maintient l'ostéotome au contact de l'os ou du cartilage et frappe l'extrémité de l'ostéotome qui n'est pas en contact avec le tissu à l'aide d'un maillet pour sectionner, fracturer ou façonner l'os ou le cartilage.

Aujourd'hui, il n'existe pas de dispositif permettant de contrôler de manière satisfaisante et en temps réel la position et la progression de l'ostéotome dans l'organe du patient (e.g. dans le nez du patient). L'utilisation d'un ostéotome boutonné permet au praticien de suivre la progression de l'ostéotome sous la peau, mais se révèle insuffisante pour contrôler la position de l'ostéotome par rapport aux tissus. Le praticien s'appuie également sur le bruit généré et sur son expérience pour adapter l'intensité et le nombre d'impacts ainsi que l'angle de frappe. Cependant, cette approche est purement empirique, dépend du praticien et manque de fiabilité. Les approches de type rayons X (amplificateur de brillance) ne sont pas utilisables facilement car trop lourdes à mettre en place et ionisantes pour le patient et pour le praticien. Les approches de type IRM ne sont pas utilisables du fait du coût et de la disponibilité du matériel.

Pouvoir évaluer, lors d'une ostéotomie, la solidité du tissu au contact de l'extrémité tranchante de l'ostéotome permettrait au praticien de se faire une idée:
- de la nature de ce tissu, e.g. cartilage ou os,
- de la solidité de l'os impacté, et/ou
- de l'apparition de fissures dans l'os impacté.

Le praticien pourrait alors mieux évaluer le positionnement et la progression de l'ostéotome dans les tissus, l'intensité et le nombre d'impacts nécessaires pour fracturer l'os et le moment à partir duquel l'os est fracturé.

Dans ce contexte et, plus généralement, dans le contexte de recueil d'informations sur un matériau quelconque, un but de l'invention est de proposer un dispositif permettant d'évaluer la solidité d'un matériau sur lequel on tape au moyen d'un outil ancillaire.

EP 2 923 677 A1 divulgue un dispositif pour évaluer la solidité d'un matériau avec un outil ancillaire en définissant une fenêtre temporelle positionnée sur ledit signal de mesure.

### PRESENTATION GENERALE

L'invention concerne un dispositif pour évaluer la solidité d'un matériau comprenant: un outil ancillaire ayant des première et deuxième extrémités opposées, la deuxième extrémité ayant une forme de pointe ou de lame, un impacteur adapté pour taper sur la première extrémité de l'outil ancillaire, au moins un capteur et une unité de traitement.

L'outil ancillaire est adapté pour être placé entre un matériau et l'impacteur, avec sa deuxième extrémité au contact du matériau, et pour transmettre au matériau la force d'impaction exercée par l'impacteur.

Le capteur est apte à mesurer une grandeur parmi la force d'impaction exercée et la déformation de l'impacteur, et à fournir un signal de mesure représentant la variation temporelle de ladite grandeur lors d'un impact.

L'unité de traitement est adaptée pour calculer, à partir du signal de mesure, un indicateur représentatif de la solidité du matériau.

La solution proposée repose donc sur la mise en oeuvre d'un ou plusieurs capteurs associés à l'impacteur et délivrant un signal de mesure, l'enregistrement et l'analyse de ce signal permettant de déterminer un indicateur révélateur de la solidité du matériau. Lorsque plusieurs capteurs sont utilisés, les signaux respectivement délivrés par ces capteurs peuvent être, par exemple, moyennés ou combinés pour obtenir le signal de mesure qui sera analysé et à partir duquel l'indicateur sera calculé.

Un tel dispositif permet, par exemple, au cours d'une ostéotomie, de renseigner le praticien sur la solidité du matériau, os ou cartilage, au contact de l'ostéotome.

Dans un autre exemple, l'outil ancillaire est une aiguille destinée à être insérée dans un tissu biologique. L'analyse des signaux obtenus lors de l'impact sur l'aiguille permet au praticien d'évaluer la solidité du tissu à proximité de la deuxième extrémité, i.e. la pointe, de l'aiguille.

Outre son coût réduit, ce dispositif a l'avantage d'être simple d'utilisation. En particulier, avec ce dispositif, le geste du praticien lors de l'opération reste le même. Le praticien n'a donc pas à apprendre de nouveaux gestes et peut tirer profit de l'expérience qu'il a déjà acquise avec des outils (outil ancillaire et impacteur) conventionnels.

L'indicateur proposé correspond à la durée d'une fenêtre temporelle. Le début de cette fenêtre temporelle est défini par rapport à un instant correspondant au premier pic d'amplitude maximale du signal de mesure et la fin de cette fenêtre temporelle étant définie par rapport à un instant correspondant au deuxième pic d'amplitude maximale du signal de mesure. Il a été montré que l'indicateur ainsi calculé était corrélé à la solidité du matériau au contact de l'outil ancillaire, qui elle-même dépend de la géométrie du matériau, des propriétés mécaniques de celui-ci, de la présence de fissures dans le matériau, etc.

Dans certains modes de réalisation, l'unité de traitement détecte dans le signal de mesure le premier pic d'amplitude maximale et le pic d'amplitude maximale succédant au premier pic, ce dernier pic n'étant considéré comme le deuxième pic d'amplitude maximale que si le signal de mesure devient inférieur à une valeur limite prédéterminée entre ces deux pics (i.e. si le signal de mesure descend au-dessous de la valeur limite avant de repasser au-dessus de cette valeur pour former le deuxième pic). En particulier, la valeur limite peut être comprise entre 1 à 20% de l'amplitude maximale du premier pic. Par exemple, le pic d'amplitude maximale succédant au premier pic n'est considéré comme le deuxième pic d'amplitude maximale que si le signal de mesure passe au-dessous d'une valeur limite égale à 5% de l'amplitude maximale du premier pic.

Cette précaution permet d'éviter les erreurs de mesure liées à un phénomène de dédoublement du premier pic, qui a pu être observé dans un petit nombre de cas. Lors d'un tel phénomène, les deux pics obtenus par dédoublement du premier pic sont proches l'un de l'autre et les inventeurs ont réalisé que le signal de mesure n'avait pas le temps de diminuer significativement entre ces deux pics. Aussi, la solution consistant à vérifier que le signal de mesure a suffisamment diminué avant d'atteindre le deuxième pic d'amplitude maximale, permet d'éviter de considérer à tort le doublon du premier pic comme le deuxième pic d'amplitude maximale, et donc d'éviter une erreur de mesure de l'indicateur. Bien entendu, d'autres méthodes d'analyse du signal de mesure pourraient être envisagées pour détecter un dédoublement du premier pic et éviter des erreurs de mesure dans un tel cas.

Dans certains modes de réalisation le dispositif comprend, en outre, un système d'alerte relié à l'unité de traitement et coopérant avec celle-ci de manière à émettre un signal d'alerte lorsque l'indicateur franchit une valeur seuil prédéterminée. Cette valeur seuil peut être déterminée expérimentalement. Bien entendu, d'autres conditions relatives à l'indicateur lui-même ou à la variation de l'indicateur lors d'une série d'impacts successifs pourraient être utilisées pour déclencher une alerte, sans sortir du cadre de l'invention. Dans le cadre d'une opération chirurgicale, l'alerte peut permettre au praticien d'arrêter de frapper l'outil ancillaire avec l'impacteur.

Dans certains modes de réalisation, l'outil ancillaire est un ciseau, un poinçon ou une aiguille. En particulier, il peut s'agir d'un ciseau chirurgical ou ostéotome.

Dans certains modes de réalisation, l'impacteur a une face de frappe adaptée pour taper sur l'outil ancillaire et le capteur est un capteur de force apte à mesurer la force d'impaction et à fournir un signal de mesure représentant la variation temporelle de la force d'impaction lors d'un impact. Dans ce cas, le capteur de force est, de préférence, fixé sur la face de frappe.

Dans d'autres modes de réalisation, l'impacteur a une face de frappe adaptée pour impacter la surface d'impact, une face opposée à la face de frappe et des faces latérales s'étendant entre la face de frappe et la face opposée, et le capteur est un capteur de déformation apte à mesurer la déformation de l'impacteur et à fournir un signal de mesure représentant la variation temporelle de la déformation de l'impacteur lors d'un impact. Dans ce cas, le capteur de déformation est, de préférence, fixé sur une des faces latérales de l'impacteur.

Dans certains modes de réalisation, l'impacteur est un marteau, ou équivalent, et comprend un manche surmonté d'une tête de frappe. En particulier, l'impacteur peut avoir sensiblement la même forme et le même poids que les impacteurs couramment utilisés jusqu'à présent. Ainsi, les praticiens expérimentés sont immédiatement en mesure de manier correctement l'impacteur.

On notera que, l'impacteur exerce la force d'impaction sur le matériau par l'intermédiaire de l'outil ancillaire. En d'autres termes, la force d'impaction est exercée sur l'outil ancillaire et transmise par celui-ci au matériau.

L'invention concerne également un procédé pour évaluer la solidité d'un matériau, dans lequel:
- on fournit un dispositif tel que précédemment décrit,
- on tape sur l'outil ancillaire à l'aide de l'impacteur, l'outil ancillaire étant placé entre l'impacteur et un matériau, avec sa deuxième extrémité au contact du matériau, de manière à transmettre au matériau la force d'impaction exercée par l'impacteur,
- on calcule l'indicateur à partir du signal de mesure fourni par le capteur, et
- on évalue la solidité du matériau sur la base de l'indicateur.

L'outil ancillaire peut être, mais n'est pas nécessairement, un ostéotome. Dans ce cas, on tape avec l'impacteur sur l'ostéotome préalablement enfoncé dans le corps d'un patient (e.g. dans le nez), de manière à obtenir le signal de mesure, et on détermine, sur la base de l'indicateur, la solidité du matériau au contact de la deuxième extrémité, i.e. la lame, de l'ostéotome. On peut alors en déduire des informations sur la position de l'ostéotome dans le corps, sur l'épaisseur du matériau, sur l'état (fissuré ou non) du matériau, etc.

L'outil ancillaire peut également être une aiguille. Dans ce cas, on tape avec l'impacteur sur l'aiguille préalablement insérée dans un tissu biologique, de manière à obtenir le signal de mesure, et on détermine, sur la base de l'indicateur, la solidité du tissu au contact de la deuxième extrémité, i.e. la pointe, de l'aiguille. L'analyse des signaux obtenus lors de l'impact sur l'aiguille permet au praticien d'évaluer la solidité du tissu.

Les avantages d'un tel procédé découlent des avantages du dispositif utilisé.

Les caractéristiques et avantages précités, ainsi que d'autres, apparaîtront à la lecture de la description détaillée qui suit, d'exemples de réalisation du dispositif proposé. Cette description détaillée fait référence aux dessins annexés.

### BREVE DESCRIPTION DES DESSINS

Les dessins annexés sont schématiques et ne sont pas à l'échelle, ils visent avant tout à illustrer les principes de l'invention.
- la figure 1 est une représentation schématique d'un montage comprenant un outil ancillaire, un impacteur, un capteur, une unité de traitement et un échantillon de matériau sur lequel on tape avec l'outil ancillaire.
- la figure 2 représente schématiquement un exemple de signal obtenu à l'aide du capteur de la figure 1, lorsqu'on donne un coup sur l'échantillon.
- la figure 3 représente les valeurs de l'indicateur pour différents types de matériaux.
- la figure 4 représente la variation de l'indicateur pour trois matériaux différents, lorsque l'épaisseur de l'échantillon varie.
- la figure 5 représente deux exemples de signaux obtenus pour un même échantillon, l'un avant fracture de l'échantillon, l'autre après fracture.
- la figure 6 représente la variation de l'indicateur lors de coups successifs portés sur un même échantillon, l'un des coups provoquant une fracture dans l'échantillon.

### DESCRIPTION DETAILLEE

La figure (FIG) 1 représente un exemple de dispositif 1 pour évaluer la solidité d'un matériau. Ce dispositif 1 comprend un outil ancillaire 2 ayant une première extrémité 2A et une deuxième extrémité 2B opposée à la première extrémité 2A. La deuxième extrémité 2B a une forme de lame: elle est mince, effilée et se termine par une arrête. L'outil ancillaire 2 est, par exemple, un ciseau chirurgical, ou ostéotome, du type de ceux utilisés pour les opérations de rhinoplastie. Les résultats expérimentaux décrits plus loin ont été obtenus avec un ostéotome de 10mm de large (longueur de la lame) commercialisé par la société Zepf, Tuttlingen, Allemagne, sous la référence 32-6002-10.

Le dispositif 1 comprend également un impacteur 4 de type marteau ou équivalent, comprenant un manche 5 surmonté d'une tête de frappe 7. L'outil ancillaire 2 et l'impacteur 4 sont adaptés pour pouvoir frapper, ou impacter, la première extrémité 2A avec l'impacteur 4. L'impacteur 4 est, par exemple, un maillet chirurgical 5. Les résultats expérimentaux décrits plus loin ont été obtenus avec un maillet chirurgical de 260g commercialisé par la société Zepf, Tuttlingen, Allemagne, sous la référence 32-6906-26.

L'impacteur 4 est équipé d'un capteur de force 12 apte à mesurer la force d'impaction exercée par l'impacteur 4 à fournir un signal de mesure représentant la variation temporelle de la force d'impaction lors d'un impact. Le capteur 12 est apte à convertir en un signal électrique exploitable la force d'impaction appliquée sur l'ensemble formé par l'outil outil ancillaire 2 et le matériau 8 lors de chaque frappe. Il s'agit, par exemple, d'un capteur à jauge ou d'un capteur piézo-électrique connecté, selon un montage approprié, à une unité de traitement 30. Dans l'exemple représenté, le capteur de force 12 est fixé sur la face de frappe 7A de la tête 7 de l'impacteur 4. Par exemple, il est vissé au centre de cette face de frappe 7A. En variante, le capteur de force 12 peut être positionné au niveau de la première extrémité 2A de l'outil ancillaire. Les résultats expérimentaux décrits plus loin ont été obtenus avec un capteur de force piézoélectrique commercialisé par la société PCB Piezotronics, Depew, NY, États-Unis, sous la référence 208C04, avec une gamme de mesure courant jusqu'à 4.45 kN en compression.

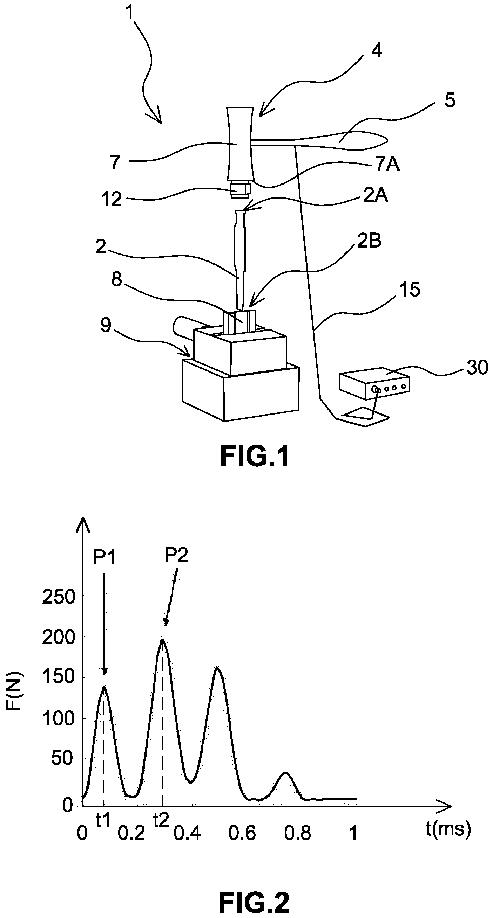

Le dispositif comprend également une unité de traitement 30 couplée au capteur 12 et configurée pour traiter les signaux de mesure délivrés par le capteur 12. Cette unité de traitement 30 comprend, par exemple, un microcontrôleur. L'unité de traitement 30 peut être logée dans un boîtier externe. En variante, l'unité de traitement 30 peut être intégrée à l'impacteur 4. Selon une autre variante, l'unité de traitement 30 peut être formée d'éléments séparés comme un micro-ordinateur relié à un module d'acquisition de données lui-même relié au capteur 12. La liaison entre le capteur 12 et l'unité de traitement 30 est, dans l'exemple de la FIG 1, réalisée de manière filaire au moyen d'un câble 15. En variante, la transmission des signaux de mesure fournis par le capteur 12 peut se faire au moyen d'une liaison sans fil, auquel cas le capteur 12 est équipé d'une antenne ou équivalent, permettant la transmission des signaux de mesure vers l'unité de traitement 30.

Les résultats expérimentaux présentés ci-après ont été obtenus avec une unité de traitement 30 comprenant un module d'acquisition "LabVIEW" commercialisé sous la référence NI 9234 par la société National Instruments, Austin, TX, Etats-Unis, avec une fréquence d'échantillonnage supérieure à 50 kHz. Dans l'exemple, la fréquence d'échantillonnage était de 51.2 kHz et la résolution de 24 bits. Ce module a été utilisé pour enregistrer l'évolution de la force exercée par l'impacteur 4 au cours du temps. Les données sont transférées vers un ordinateur via une interface graphique "LabVIEW" pour une durée de mesure de 10 ms ou moins.

L'outil ancillaire 2 et l'impacteur 4 sont utilisés pour taper sur un échantillon de matériau 8. Pour ce faire, la deuxième extrémité 2B de l'outil ancillaire 2 est placée au contact de l'échantillon de matériau 8 et l'outil ancillaire 2 est impacté par l'impacteur 4 de sorte que la force d'impaction exercée par l'impacteur 4 soit transmise au matériau 8 par l'outil ancillaire 2. Pour les tests, l'échantillon est immobilisé, par exemple en étant serré dans un étau 9. Lors de chaque impact, le capteur 12 mesure la force d'impaction exercée et fournit un signal de mesure représentant la variation temporelle de cette force pendant l'impact. On considère que l'impact commence à partir de l'instant où l'impacteur 4, l'outil ancillaire 2 et le matériau 8 sont au contact les uns des autres et dure pendant un certain laps de temps après cet instant. Ce laps de temps est inférieur à 50 ms. Un exemple de signal fourni par le capteur 12 est représenté sur la FIG. 2 et décrit plus loin.

Les inventeurs ont eu l'idée de s'intéresser à un tel signal de mesure et ont mis en évidence le fait que ce signal portait des informations sur la solidité du matériau 8. En particulier, les inventeurs ont réussi à déterminer, à partir du signal de mesure recueilli, un indicateur représentatif de cette solidité, comme expliqué ci-après.

Pour essayer d'expliquer le lien entre le signal de mesure recueilli et la solidité du matériau 8, l'explication suivante peut être avancée. L'impacteur 4 exerce sur l'échantillon de matériau 8, via l'outil ancillaire 2, une force d'impaction qui est à l'origine de modes de vibration dans l'ensemble du système formé par l'impacteur 4, le capteur 12, l'outil ancillaire 2 et le matériau 8 lorsque ces éléments sont tous en contact lors de l'impact. Ces modes de vibration dépendent de la solidité du matériau 8. Schématiquement, plus le matériau 8 est solide, plus le système est rigide et plus les fréquences de résonnance des modes de vibration sont élevées.

La FIG 2 est un graphique représentant schématiquement un exemple de signal obtenu à l'aide du capteur de force 12 lors d'un impact sur un échantillon. Le temps (t) en millisecondes (ms) est reporté en abscisse, la force (F) en Newtons (N), mesurée par le capteur 12, est reportée en ordonnée.

Le signal de la FIG 2 correspond à un impact ou coup. L'impact est porté au temps t = 0. Le premier pic d'amplitude maximale P1 apparaît rapidement (i.e. moins de 0.1 milliseconde après) au temps t1. Le deuxième pic d'amplitude maximale apparaît jute après le premier pic P1 (i.e. un peu plus de 0.1 milliseconde après) au temps t2. Les inventeurs ont mis en évidence le fait que cette durée (t2 - t1) entre les premier et deuxième pics P1, P2 était un indicateur IN fiable et pertinent pour refléter la solidité du matériau.

Les tests décrits ci-dessous, en référence aux figures annexées, illustrent le lien entre l'indicateur IN et la solidité du matériau. Les matériaux testés étaient du contreplaqué ordinaire de bouleau (3 plis), du polycarbonate (Nudec, Barcelone, Espagne), une résine en polyuréthane (SmoothCast 300, Smooth-On, Eaton, PA, États-Unis) et de l'os artificiel "ORTHObone" (3B Scientific, Hamburg, Allemagne) de trois densités différentes (ORTHObone 10 PCF, 20PCF et 30 PCF). Ces matériaux sont notés respectivement "PW", "PC", "RS", "O1", "O2" et "O3". Chaque matériau a été découpé en échantillons de 3 x 4.5 cm d'épaisseurs variables comprises entre 2 et 8mm, pour un total de 100 échantillons. Chaque échantillon avait ainsi la forme d'une plaquette. Chaque plaquette a été placée à la verticale et serrée dans l'étau 9, l'arête tranchante de l'extrémité 2B de l'outil ancillaire 2 étant perpendiculaire à la plaquette, comme représenté sur la FIG 1. En d'autres termes, la direction de l'épaisseur de l'échantillon était parallèle à l'arrête de la lame de l'outil ancillaire 2. Ainsi, l'épaisseur de l'échantillon correspond, dans cet exemple, à la longueur de la zone de contact entre l'échantillon et l'arête tranchante de l'extrémité 2B en forme de lame de l'outil ancillaire 2. Par ailleurs, la longueur (i.e. la longueur totale) de l'arête tranchante est supérieure à la longueur de la zone de contact entre l'échantillon et l'arête tranchante.

L'épaisseur de chaque échantillon a été mesurée à l'aide d'un pied à coulisse. Les propriétés mécaniques, la moyenne et l'écart-type de la distribution des épaisseurs pour les six matériaux testés sont donnés dans le tableau ci-dessous où "E" désigne le module d'Young en MPa, "tan delta" désigne le facteur d'amortissement ou facteur de perte correspondant à la viscoélasticité du matériau, "dₘ" et "sigma d", en mm, désignent respectivement la moyenne et l'écart-type de la distribution des épaisseurs pour chaque matériau, "d-" désigne l'épaisseur minimum en mm, "d+" désigne l'épaisseur maximum en mm et N le nombre d'échantillons pour chaque matériau.

| Material | *E* (MPa) | tan *δ* | *dₘ* (mm) | *σ_{d}* (mm) | *d*⁻ (mm) | *d*⁺ (mm) | *N* |
|---|---|---|---|---|---|---|---|
| PW | 2400 | 0.01 | 4.7 | 1.5 | 3.0 | 6.0 | 11 |
| PC | 1300 | 0.02 | 4.9 | 1.2 | 3.0 | 6.0 | 20 |
| RS | 500 | 0.03 | 4.7 | 1.5 | 2.4 | 6.8 | 17 |
| O1 | 41 | 0.03 | 4.4 | 1.4 | 2.4 | 7.1 | 18 |
| O2 | 250 | 0.03 | 4.1 | 1.5 | 2.5 | 7.1 | 15 |
| O3 | 380 | 0.03 | 4.5 | 1.2 | 2.1 | 5.8 | 19 |

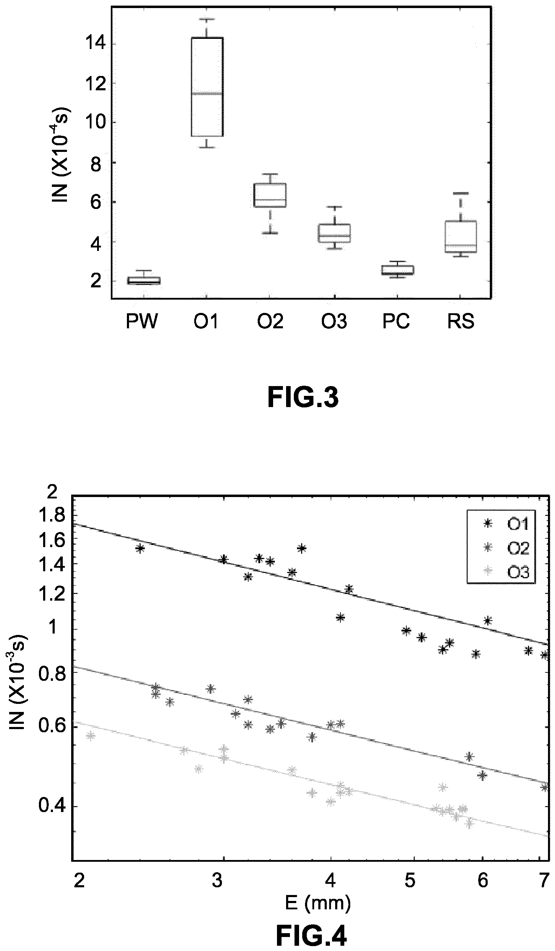

La FIG 3 est un graphique représentant sous forme de boîte à moustaches la distribution de l'indicateur IN pour les différents types de matériau 8. L'indicateur IN est exprimé en dixième de milliseconde (10⁻⁴ s) et reporté en ordonnée. Le type de matériau 8 est reporté en abscisse. La barre horizontale centrale indique la médiane et les côtés supérieur et inférieur de la boîte indiquent les quartiles supérieur et inférieur. Les moustaches s'étendent jusqu'aux valeurs extrêmes de la distribution. Les valeurs de la FIG 3 sont groupées selon les types de matériau seulement, sans aucune considération sur l'épaisseur des échantillons. Cependant, comme discuté plus loin, l'épaisseur a un impact sur l'indicateur IN et induit inévitablement une part de la dispersion de valeurs observée.

La FIG 3 montre que le type de matériau a une influence significative sur l'indicateur IN. De manière générale, plus le module d'Young du matériau est élevé, et donc plus le matériau est rigide, plus l'indicateur IN diminue. Les tests de Tukey-Kramer indiquent que les résultats obtenus sont significativement différents d'un type de matériau à l'autre, excepté pour deux couples de matériaux différents: le couple PW et PC, et le couple O3 et RS.

La FIG 4 représente la variation de l'indicateur IN pour trois matériaux différents, à savoir les os artificiels O1, O2 et O3, lorsque l'épaisseur de l'échantillon varie. La FIG 4 montre que l'épaisseur de l'échantillon a une influence significative sur l'indicateur IN. De manière générale, plus l'épaisseur de l'échantillon est importante, plus l'indicateur IN diminue.

Les FIGS 3 et 4 montrent donc que l'indicateur IN varie en fonction de la longueur de la zone de contact entre l'échantillon et l'arête tranchante de l'outil, et en fonction du module d'Young du matériau constitutif de l'échantillon. En d'autres termes, l'indicateur IN varie en fonction de l'épaisseur de l'échantillon et de la rigidité du matériau constitutif de l'échantillon.

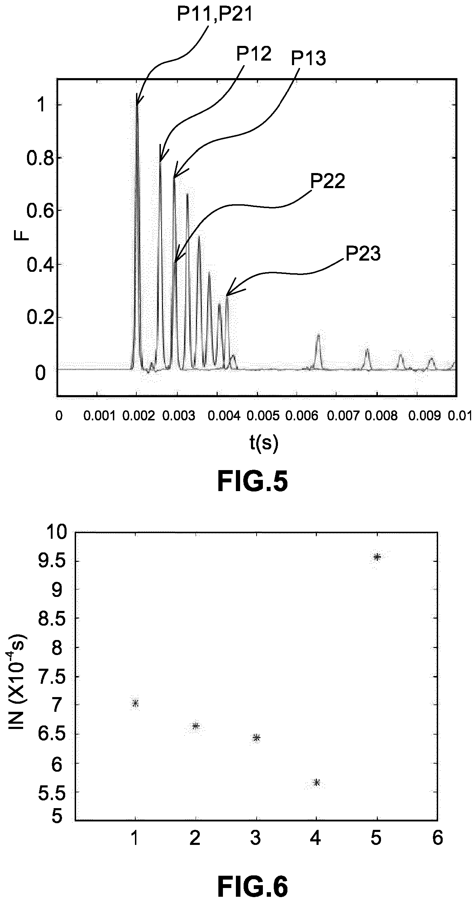

Il est à noter que la solidité de l'échantillon augmente en fonction de sa rigidité (dans le cas d'un tissu biologique) et de son épaisseur. En outre, les FIGS 5 et 6, décrites plus loin, montrent que l'indicateur IN varie lorsqu'une fissure apparait dans l'échantillon. Au final, on comprend que l'indicateur IN est lié, de manière générale, à la solidité du matériau de l'échantillon: plus ce matériau est solide du fait de sa rigidité, de son épaisseur importante ou de l'absence de fissure, plus l'indicateur IN est faible. A l'inverse, plus le matériau est fragile, du fait de son manque de rigidité, de sa faible épaisseur ou de la présence de fissure, plus l'indicateur IN est élevé. On comprend que, dans le présent exposé, la notion de "solidité" du matériau doit s'entendre comme une combinaison de l'épaisseur du matériau, de sa rigidité matérielle et de la présence ou non de fissure.

La FIG 5 est un graphique avec, en ordonnée, la force "F" mesurée par le capteur exprimée en unité normalisée et, en abscisse, le temps "t" en secondes. La FIG 5 représente deux exemples de signaux obtenus pour un même échantillon, l'un avant fracture de l'échantillon, l'autre après fracture. Les trois premiers pics du signal avant fracture sont notés P11, P12, P13 et les trois premiers pics du signal après fracture sont notés P21, P22, P23. On constate que l'indicateur IN, qui correspond à la durée entre les deux premiers pics P11, P12 ou P21, P22, augmente significativement une fois l'échantillon fracturé, i.e. une fois qu'une fissure est apparue dans l'échantillon.

La FIG 6 est un graphique représentant l'évolution de l'indicateur IN en fonction du nombre d'impacts portés sur l'échantillon. Le nombre d'impacts (de 1 à 5) est reporté en abscisse. L'indicateur IN, exprimé en dixièmes de milliseconde (ms), est reporté en ordonnée. Dans cet exemple, on a constaté l'apparition d'une fissure dans l'échantillon entre les impacts numéro 4 et 5. On voit que l'indicateur IN augmente significativement du fait de l'apparition de cette fissure.

Sur la base de tests équivalents à celui de la FIG 6, il est possible, pour un même matériau ayant sensiblement la même épaisseur, de déterminer une valeur seuil S1 pour l'indicateur IN au-delà de laquelle l'apparition d'une fissure dans le matériau a nécessairement eu lieu. Dans l'exemple de la FIG 6, la valeur seuil S1 pourrait être choisie égale à 8.5 × 10⁻⁴ s. Bien entendu, il ne s'agit là que d'un exemple.

Une fois déterminée, la valeur seuil S1 peut être utilisée pour paramétrer le dispositif 1. Cette valeur seuil S1 est, par exemple, enregistrée dans la mémoire de l'unité de traitement 30. En outre, le dispositif 1 peut comprendre un système d'alerte (non représenté) pour émettre un signal d'alerte (par exemple, un signal sonore, visuel et/ou tactile). Le système d'alerte est relié à l'unité de traitement 30 et coopère avec celle-ci pour alerter le praticien lorsque l'apparition d'une fissure est détectée sur la base de l'indicateur IN, par exemple dès que l'indicateur IN passe au dessus de la valeur seuil S1. Un autre mode de réalisation peut consister à détecter une brusque variation de l'indicateur IN entre deux impacts successifs, qui est susceptible d'être liée à l'apparition d'une fissure.

A titre d'exemple, le dispositif 1 qui vient d'être décrit peut être utilisé lors d'une ostéotomie. Cette opération chirurgicale consiste à utiliser comme outil ancillaire 2 un ostéotome, et à introduire cet ostéotome dans le nez d'un patient afin d'induire une fracture en bois vert dans l'os et le cartilage. Ceci permet de modifier par la suite la forme du nez. Cette fracture est réalisée en impactant l'ostéotome à l'aide de l'impacteur 4. Cette intervention est considérée comme assez délicate car elle se pratique la plupart du temps en introduisant l'ostéotome sous la peau, ce qui rend difficile de connaître la position réelle de l'arrête tranchante. En utilisant le dispositif proposé, le chirurgien va pouvoir, notamment, évaluer la position de cette arrête tranchante.

En effet, l'évaluation de la solidité des tissus au contact de l'arrête tranchante de l'ostéotome, via l'indicateur IN, permet au praticien de savoir si cette arrête tranchante se situe dans le cartilage ou dans l'os du nez, ces tissus n'ayant pas la même solidité Le praticien peut alors adapter l'énergie délivrée lors des impacts. Si l'arrête tranchante se situe dans l'os, le praticien doit impacter plus fortement, alors que dans le cartilage, il doit être plus mesuré dans ses impacts afin de ne pas induire de blessures supplémentaires.

Le suivi de l'indicateur IN, en particulier le dépassement de la valeur seuil S1 ou une brusque augmentation de IN entre deux impacts successifs, permet également d'alerter le praticien de l'apparition d'une fissure dans l'os. Le praticien peut alors s'arrêter d'impacter, la présence de fissures non contrôlées pouvant mener à des complications post-opératoires. On comprend donc que le suivi de l'indicateur IN permet d'évaluer la solidité de l'os et, plus généralement, du matériau testé, en évitant l'apparition de fissures non contrôlées dans ce matériau. En ce sens, l'évaluation réalisée est non destructive.

Enfin, le suivi de l'indicateur IN permet d'évaluer l'épaisseur de l'os au contact de l'arrête tranchante, car cette épaisseur augmente progressivement jusqu'à l'os frontal, à l'endroit précis où les lignes de fracture se rejoignent et où le chirurgien doit s'arrêter d'impacter pour éviter de perforer l'os frontal. L'augmentation soudaine de l'épaisseur de l'os indique le début de l'os frontal et se traduit par une diminution soudaine de l'indicateur IN. Il est important à ce stade que le praticien arrête d'impacter l'ostéotome car il existe un risque de fracture complète du nez, qui peut mener à l'endommagement des vaisseaux et à des complications graves.

L'exemple d'utilisation qui vient d'être décrit est donné à titre illustratif et non limitatif, une personne du métier pouvant facilement tirer profit du dispositif et de l'indicateur proposé par les inventeurs dans d'autres applications qu'une ostéotomie, y compris dans des applications non chirurgicales.

En outre, l'exemple qui vient d'être décrit met en oeuvre un capteur de force 12. Selon un autre exemple (non représenté), il est possible d'utiliser un capteur de déformation apte à fournir un signal de mesure représentant la variation temporelle de la déformation de l'impacteur 4 lors d'un impact. Un tel capteur de déformation est apte à convertir en un signal électrique exploitable la déformation de la tête de frappe 7 de l'impacteur 4. Dans ce cas, au lieu d'être situé sur la face de frappe 7a comme le capteur de force 12, le capteur de déformation est positionné sur une des faces latérales de la tête de frappe 7. En l'occurrence, le capteur de déformation peut être positionné sur la face latérale s'étendant parallèlement à la direction de l'axe du manche 5. Plus précisément, vu de côté, le capteur de déformation peut être fixé sur la partie avant de la face latérale, entre la face de frappe 7A et l'axe du manche 13. L'avant et l'arrière sont définis ici par rapport au mouvement de frappe de l'impacteur 4. Le capteur de déformation est fixé sur la tête de frappe 7, par exemple par collage ou tout autre moyen de fixation approprié, de sorte que la déformation de la tête de frappe 7 provoque la déformation du capteur. Le capteur est, par exemple, un capteur à jauge comprenant un élément de mesure élastique dont la déformation est d'abord convertie en une variation de résistance électrique de la jauge, pour générer ensuite un signal de sortie électrique. En variante, il peut s'agir d'un capteur piézo-électrique reposant sur les propriétés piézo-électriques d'un matériau (e.g. du quartz ou des céramiques synthétiques) qui génère une charge électrique lorsqu'il se déforme.

Le signal de mesure fourni par un tel capteur de déformation et représentant la variation temporelle de la déformation de l'impacteur lors d'un impact présente également des premier et deuxième pics d'amplitude maximale. Le laps de temps séparant ces deux pics s'avère également être un indicateur fiable et pertinent pour évaluer le niveau de contact entre l'implant et l'os récepteur.

Par ailleurs, l'exemple qui vient d'être décrit met en oeuvre un outil ancillaire ayant une extrémité en forme de lame. Selon un autre exemple (non représenté), il est possible d'utiliser un outil ancillaire ayant une extrémité en forme de pointe comme un poinçon ou une aiguille.

Enfin, les différentes caractéristiques des modes ou exemples de réalisation décrits dans le présent exposé peuvent être considérées isolément ou être combinées entre elles. Lorsqu'elles sont combinées, ces caractéristiques peuvent l'être comme décrit ci-dessus ou différemment, l'invention ne se limitant pas aux combinaisons spécifiques précédemment décrites. En particulier, sauf précision contraire ou incompatibilité technique, une caractéristique décrite en relation avec un mode ou exemple de réalisation peut être appliquée de manière analogue à un autre mode ou exemple de réalisation.

## Revendications

1. Dispositif pour évaluer la solidité d'un matériau comprenant:
- un outil ancillaire (2) ayant des première et deuxième extrémités opposées (2A, 2B),
- un impacteur (4) adapté pour taper sur la première extrémité (2A) de l'outil ancillaire (2),
- au moins un capteur (12), et
- une unité de traitement (30),
dans lequel l'outil ancillaire (2) est adapté pour être placé entre un matériau (8) et l'impacteur (4), avec sa deuxième extrémité (2B) au contact du matériau (8), et pour transmettre au matériau (8) la force d'impaction exercée par l'impacteur (4),
dans lequel le capteur (12) est apte à mesurer une grandeur parmi la force d'impaction exercée et la déformation de l'impacteur, et à fournir un signal de mesure représentant la variation temporelle de ladite grandeur lors d'un impact,
dans lequel l'unité de traitement (30) est adaptée pour calculer, à partir du signal de mesure, un indicateur (IN) représentatif de la solidité du matériau (8), et
dans lequel l'indicateur (IN) correspond à la durée d'une fenêtre temporelle, le début de cette fenêtre temporelle étant défini par rapport à un instant (t1) correspondant au premier pic (P1) d'amplitude maximale du signal de mesure,
le dispositif étant **caractérisé en ce que** la fin de ladite fenêtre temporelle est définie par rapport à un instant (t2) correspondant au deuxième pic d'amplitude maximale (P2) du signal de mesure, et **en ce que** la deuxième extrémité (2B) de l'outil ancillaire (2) a une forme de pointe ou de lame.

2. Dispositif selon la revendication 1, dans lequel l'unité de traitement (30) détecte dans le signal de mesure le premier pic (P1) d'amplitude maximale et le pic d'amplitude maximale succédant au premier pic (P1), ce dernier pic n'étant considéré comme le deuxième pic d'amplitude maximale (P2) que si l'amplitude du signal devient inférieure à une valeur limite prédéterminée entre les deux pics.

3. Dispositif selon la revendication 2, dans lequel la valeur limite prédéterminée est comprise entre 1 et 20 % de l'amplitude maximale du premier pic (P1).

4. Dispositif selon l'une quelconque des revendications 1 à 3 comprenant, en outre, un système d'alerte relié à l'unité de traitement (30) et coopérant avec celle-ci de manière à émettre un signal d'alerte lorsque l'indicateur (IN) franchit une valeur seuil (S1) prédéterminée.

5. Dispositif selon l'une quelconque des revendications 1 à 3 comprenant, en outre, un système d'alerte relié à l'unité de traitement (30) et coopérant avec celle-ci de manière à émettre un signal d'alerte lorsque l'indicateur (IN) varie brusquement entre deux impacts successifs.

6. Dispositif selon l'une quelconque des revendications 1 à 5, dans lequel l'outil ancillaire (2) est un ciseau, un poinçon ou une aiguille.

7. Dispositif selon l'une quelconque des revendications 1 à 6, dans lequel l'outil ancillaire (2) est un ostéotome.

8. Dispositif selon l'une quelconque des revendications 1 à 7, dans lequel le capteur (12) est fixé sur l'impacteur (4) ou sur l'outil ancillaire (2).

9. Dispositif selon l'une quelconque des revendications 1 à 8, dans lequel l'impacteur (4) a une face de frappe (7A) adaptée pour taper sur l'outil ancillaire (2) et dans lequel le capteur (12) est un capteur de force fixé sur la face de frappe (7A).

10. Dispositif selon l'une quelconque des revendications 1 à 8, dans lequel l'impacteur (4) a une face de frappe (7A) adaptée pour taper sur l'outil ancillaire (2), une face opposée à la face de frappe et des faces latérales s'étendant entre la face de frappe et la face opposée, et dans lequel le capteur est un capteur de déformation fixé sur une des faces latérales de l'impacteur (4).

11. Dispositif selon l'une quelconque des revendications 1 à 10, dans lequel la deuxième extrémité (2B) de l'outil ancillaire (2) a la forme d'une lame, et dans lequel la longueur de l'arête tranchante de cette lame est supérieure à la longueur de la zone de contact entre le matériau (8) et l'arête tranchante.

## Patentansprüche

1. Vorrichtung zur Beurteilung der Festigkeit eines Werkstoffs, aufweisend:
- ein Hilfswerkzeug (2) mit einem ersten und einem gegenüberliegenden zweiten Ende (2A, 2B),
- einen Schlagkörper (4), der so beschaffen ist, dass er auf das erste Ende (2A) des Hilfswerkzeugs (2) klopft,
- mindestens einen Sensor (12), und
- eine Verarbeitungseinheit (30),
wobei das Hilfswerkzeug (2) dazu ausgebildet ist, zwischen einem Werkstoff (8) und dem Schlagkörper (4) angeordnet zu werden, wobei sein zweites Ende (2B) mit dem Werkstoff (8) in Kontakt steht, und um die durch den Schlagkörper (4) ausgeübte Schlagkraft auf den Werkstoff (8) zu übertragen,
wobei der Sensor (12) ausgebildet ist, die ausgeübte Schlagkraft oder die Verformung des Schlagkörpers zu messen und ein Messsignal zu liefern, das die zeitliche Veränderung der genannten Größe nach einem Schlag wiedergibt,
wobei die Verarbeitungseinheit (30) dazu ausgebildet ist, aus dem Messsignal einen Indikator (IN) zu berechnen, der die Festigkeit des Werkstoffs (8) wiedergibt, und
wobei der Indikator (IN) der Dauer eines Zeitfensters entspricht, wobei der Beginn dieses Zeitfensters in Bezug auf einen Zeitpunkt (11) definiert ist, der dem ersten Scheitelpunkt (P1) der maximalen Amplitude des Messsignals entspricht,
wobei die Vorrichtung **dadurch gekennzeichnet ist, dass** das Ende des Zeitfensters in Bezug auf einen Zeitpunkt (t2) definiert ist, der dem zweiten Scheitelpunkt (P2) der maximalen Amplitude des Messsignals entspricht, und dass das zweite Ende (2B) des Hilfswerkzeugs (2) die Form einer Spitze oder einer Klinge hat.

2. Vorrichtung nach Anspruch 1, wobei die Verarbeitungseinheit (30) im Messsignal den ersten Scheitelpunkt (P1) mit maximaler Amplitude und den auf den ersten Scheitelpunkt (P1) folgenden zweiten Scheitelpunkt mit maximaler Amplitude detektiert, wobei der letztgenannte Scheitelpunkt nur dann als zweiter Scheitelpunkt mit maximaler Amplitude (P2) betrachtet wird, wenn die Signalamplitude zwischen den beiden Scheitelpunkten unter einen vorbestimmten Grenzwert fällt.

3. Vorrichtung nach Anspruch 2, wobei der vorbestimmte Grenzwert zwischen 1 und 20 % der maximalen Amplitude des ersten Scheitelpunkts (P1) liegt.

4. Vorrichtung nach einem der Ansprüche 1 bis 3, die ferner ein Warnsystem aufweist, das mit der Verarbeitungseinheit (30) verbunden ist und mit dieser so zusammenarbeitet, dass es ein Warnsignal ausgibt, wenn der Indikator (IN) einen vorbestimmten Schwellenwert (S1) überschreitet.

5. Vorrichtung nach einem der Ansprüche 1 bis 3, die ferner ein Warnsystem aufweist, das mit der Verarbeitungseinheit (30) verbunden ist und mit dieser so zusammenarbeitet, dass es ein Warnsignal ausgibt, wenn sich der Indikator (IN) zwischen zwei aufeinanderfolgenden Schlägen abrupt ändert.

6. Vorrichtung nach einem der Ansprüche 1 bis 5, wobei das Hilfswerkzeug (2) ein Meißel, ein Stempel oder eine Nadel ist.

7. Vorrichtung nach einem der Ansprüche 1 bis 6, wobei das Hilfswerkzeug (2) ein Osteotom ist.

8. Vorrichtung nach einem der Ansprüche 1 bis 7, wobei der Sensor (12) an dem Schlagkörper (4) oder an dem Hilfswerkzeug (2) befestigt ist.

9. Vorrichtung nach einem der Ansprüche 1 bis 8, wobei der Schlagkörper (4) eine Fläche (7A) aufweist, die zum Schlagen auf das Hilfswerkzeug (2) ausgebildet ist, und wobei der Sensor (12) ein Kraftsensor ist, der an der Schlagfläche (7A) befestigt ist.

10. Vorrichtung nach einem der Ansprüche 1 bis 8, wobei der Schlagkörper (4) eine Schlagfläche (7A), die zum Schlagen auf das Hilfswerkzeug (2) ausgebildet ist, eine der Schlagfläche gegenüberliegende Fläche und Seitenflächen aufweist, die sich zwischen der Schlagfläche und der gegenüberliegenden Fläche erstrecken, und wobei der Sensor ein Deformationssensor ist, der an einer der Seitenflächen des Schlagkörpers (4) befestigt ist.

11. Vorrichtung nach einem der Ansprüche 1 bis 10, wobei das zweite Ende (2B) des Hilfswerkzeugs (2) die Form einer Klinge hat, und wobei die Länge der Schneidkante dieser Klinge größer ist als die Länge des Kontaktbereichs zwischen dem Werkstoff (8) und der Schneidkante.

## Claims

1. A device for assessing the solidity of a material comprising:
- an ancillary tool (2) having opposite first and second ends (2A, 2B),
- an impactor (4) suitable for striking the first end (2A) of the ancillary tool (2),
- at least one sensor (12), and
- a processing unit (30),
wherein the ancillary tool (2) is adapted for being placed between a material (8) and the impactor (4), with its second end (2B) in contact with the material (8), and for transmitting the impact force generated by the impactor (4) to the material (8),
wherein the sensor (12) is capable of measuring a quantity from among the impact force generated and the deformation of the impactor, and of supplying a measurement signal representing the variation in time of said quantity upon an impact,
wherein the processing unit (30) is adapted for calculating, from the measurement signal, an indicator (IN) representative of the solidity of the material (8), and
wherein the indicator (IN) corresponds to the duration of a time window, the start of this time window being defined with respect to an instant (t1) corresponding to the first peak (P1) of maximum amplitude of the measurement signal,
the device being **characterized in that** the end of said time window is defined with respect to an instant (t2) corresponding to the second peak of maximum amplitude (P2) of the measurement signal, and **in that** the second end (2B) of the ancillary tool (2) is in the form of a point or blade.

2. The device as claimed in claim 1, wherein the processing unit (30) detects, in the measurement signal, the first peak (P1) of maximum amplitude and the peak of maximum amplitude succeeding the first peak (P1), this latter peak being considered as the second peak of maximum amplitude (P2) only if the amplitude of the signal becomes lower than a predetermined limit value between the two peaks.

3. The device as claimed in claim 2, wherein the predetermined limit value lies between 1 and 20% of the maximum amplitude of the first peak (P1).

4. The device as claimed in any one of claims 1 to 3 further comprising an alert system linked to the processing unit (30) and cooperating therewith so as to emit an alert signal when the indicator (IN) crosses a predetermined threshold value (S1).

5. The device as claimed in any one of claims 1 to 3 further comprising an alert system linked to the processing unit (30) and cooperating therewith so as to emit an alert signal when the indicator (IN) significantly varies between two successive impacts.

6. The device as claimed in any one of claims 1 to 5, wherein the ancillary tool (2) is a chisel, a punch or a needle.

7. The device as claimed in any one of claims 1 to 6, wherein the ancillary tool (2) is an osteotome.

8. The device as claimed in any one of claims 1 to 7, wherein the sensor (12) is fixed to the impactor (4) or to the ancillary tool (2).

9. The device as claimed in any one of claims 1 to 8, wherein the impactor (4) has a strike face (7A) adapted for striking the ancillary tool (2) and wherein the sensor (12) is a force sensor fixed to the strike face (7A).

10. The device as claimed in any one of claims 1 to 9, wherein the impactor (4) has a strike face (7A) adapted for striking the ancillary tool (2), a face opposite the strike face and lateral faces extending between the strike face and the opposite face, and wherein the sensor is a deformation sensor fixed to one of the lateral faces of the impactor (4).

11. The device as claimed in any one of claims 1 to 10, wherein the second end (2B) of the ancillary tool (2) has the form of a blade, and wherein the length of the cutting edge of this blade is greater than the length of the zone of contact between the material (8) and the cutting edge.
